# EUROPEAN PATENT SPECIFICATION

(11) **EP 2 322 120 B1**
(45) Date of publication and mention of the grant of the patent: **24.04.2013**
(21) Application number: 09817670.4
(22) Date of filing: 18.09.2009
(51) Int. Cl.: A61F 2/82

(54) **STENT DELIVERY SYSTEM**
STENTTRÄGERSYSTEM
SYSTÈME DE MISE EN PLACE D'UNE ENDOPROTHÈSE

(30) Priority: 30.09.2008 JP 2008254742
(43) Date of publication of application: 18.05.2011
(73) Proprietor: Terumo Kabushiki Kaisha, Tokyo 151-0072 (JP)
(72) Inventor: SUGIMOTO Ryota, Ashigarakami-gun Kanagawa 259-0151 (JP); KITAOKA Takashi, Ashigarakami-gun Kanagawa 259-0151 (JP)
(74) Representative: Prüfer & Partner GbR European Patent Attorneys
(86) International application number: PCT/JP2009/066448
(87) International publication number: WO 2010/038634

(56) References cited:
- WO-A1-2006/037086
- WO-A2-02/083038
- WO-A2-2005/032411
- JP-A- 4 075 665
- JP-A- 2001 137 354
- JP-A- 2004 167 239
- JP-A- 2006 055 330
- JP-A- 2006 346 350
- US-B1- 6 607 539

## Description

### Technical Field

The present invention relates to a stent delivery system for use in improvement of a stenosed or occluded lesion generated in a living body lumen such as blood vessel, bile duct, trachea, esophagus, urethra, etc.

### Background Art

A stent for placement (indwelling) in living bodies is generally a tubular medical device which, for treatment of various diseases generated by stenosis or occlusion of a blood vessel or other living body lumen, is placed (put indwelling) in the stenosed or occluded lesion so as to dilate the lesion and secure the lumen at the lesion.
The following description will be made by taking a blood vessel as an example, which is not to be construed as limitative.

The stent is a body which, for insertion from the outside into the inside of a living body, is small in diameter at the time of insertion, and is expanded at the target stenosed or occluded lesion so as to be enlarged in diameter and to maintain the lumen at the lesion as it is.
In general, stents are cylindrical bodies obtained by processing of metallic wires or metallic pipe. A stent is mounted to a catheter or the like in a radially reduced state, is inserted into a living body, and is expanded at a target lesion by some method, to be fixed in close contact with the inner wall of the lumen at the lesion, thereby maintaining the lumen shape. The stents are classified by function and placement method into self-expandable stents and balloon-expandable stents. A balloon-expandable stent is a stent which itself does not have a self-expanding function. The balloon-expandable stent is used by a method in which the stent mounted on a balloon is inserted into a target lesion, and thereafter the balloon is dilated to expand (plastically deform) the stent by the expanding force of the balloon, thereby being fixed in close contact with the inner surface of the target lumen. This type of stents needs the stent-expanding operation as above-mentioned. On the other hand, a self-expandable stent is a stent which itself has a self-expanding function. The self-expandable stent is used by a method in which the stent in a radially contracted state is inserted into a living body, and is released from the contracted state at a target lesion so as to return into its original expanded state, thereby being fixed in close contact with the inner wall of the lumen at the lesion and maintaining the lumen shape.

The purpose of the placement of a stent at present is to return a blood vessel stenosed for some reason to its original open (patent) state. In most cases, the stents are mainly for preventing or reducing the risk or extent of restenosis which might occur after such a procedure as PTCA. In recent years, for suppressing the probability of restenosis more securely, drug-eluting stents with such a drug as immunosuppressor or carcinostatic loaded thereon are used, and the effect thereof is generally known.
Most of the self-expandable stents are used in peripheral regions such as inferior-limb blood vessels and carotid arteries. There are self-expandable stents which have, for example, the form as shown in JP-T Hei 11-505441 (Patent Document 1).

Patent Document 1: JP-T Hei 11-505441

### Disclosure of Invention

### Technical Problem

In the stent delivery system using a self-expandable stent as in Patent Document 1, the self-expanding property of the stent makes difficult the positioning at the time of placement of the stent, as compared with a balloon-expandable stent. In addition, there may arise a jumping phenomenon in which the stent jumps out of the dilator, and, if this phenomenon occurs, the stent would be arranged at a position deviated from a planned arrangement position. Besides, in a stent placing procedure, re-adjustment of the placing position may in some cases be needed after the stent is discharged to a certain extent. In such cases as described in Patent Document 1, however, it is difficult to re-contain the stent into the stent delivery system.

Accordingly, it is an object of the present invention to provide a stent delivery system using a self-expandable stent, wherein jumping of the stent
US 6,607,539 B1 discloses a graft delivery system comprising a graft release system having a complex entanglement arrangement in which bindings are looped around a graft, the bindings are further threaded through resistive wire loops arranged laterally to the graft, and the ends of the bindings are pulled tight to collapse the graft and stitched to graft and fixed by a knot. Upon application of voltage, the resistive wire loops burn through the bindings, thereby releasing the graft.
In WO 02/083038 A2, a conventional guide wire catheter is disclosed which comprises no breaking member for stent release.
WO 2005/032411 A2 discloses a stent with deflection elements in the form of e.g. eyelets. arising from the self-expanding property of the stent would not occur at the time of discharging the stent from a dilator, and the stent can again be contained into the dilator even after the stent is exposed out of the dilator to a certain extent.

### Technical Solution

A system for attaining the above object is as follows.
A stent delivery system including: a stent which is formed to be substantially cylindrical in shape, is compressed toward a center axis thereof at the time of insertion into a living body, and is capable of expanding outward when placed in the living body, to be restored into a pre-compression shape thereof; a shaft section having a guide wire lumen; and a sheath having the stent contained in a distal portion thereof, the stent being located at a position which is on the shaft section and near the distal end of the shaft section; wherein the stent delivery system includes a stent proximal portion fixing wire having one end portion and the other end portion fixed to the shaft section, and having an intermediate portion engaged with a proximal portion of the stent, and a breaking member for breaking the stent proximal portion fixing wire to release the stent from the engagement.

### Brief Description of Drawings

[FIG. 1]

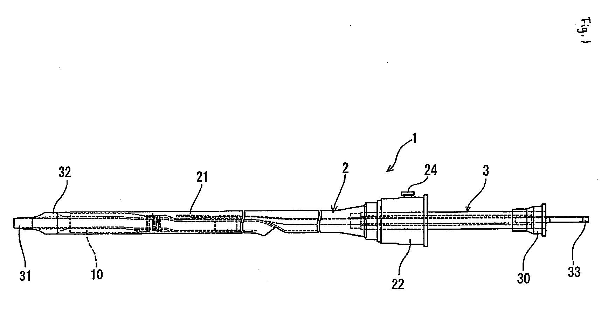

   FIG. 1 is a partly omitted front view of a stent delivery system according to an embodiment of the present invention.
[FIG. 2]

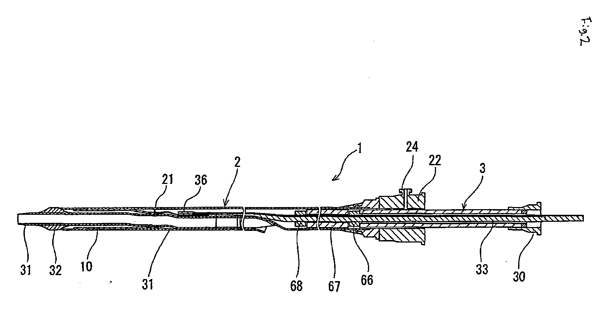

   FIG. 2 is a longitudinal sectional view of the stent delivery system shown in FIG. 1.
[FIG. 3]

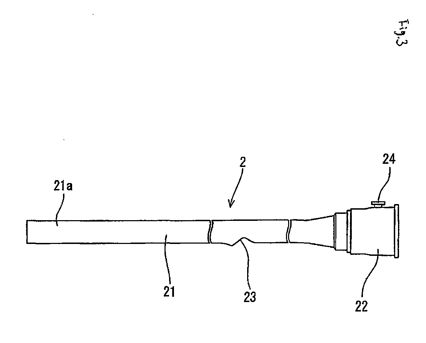

   FIG. 3 is a partly omitted front view of a sheath in the stent delivery system shown in FIG. 1.
[FIG. 4]

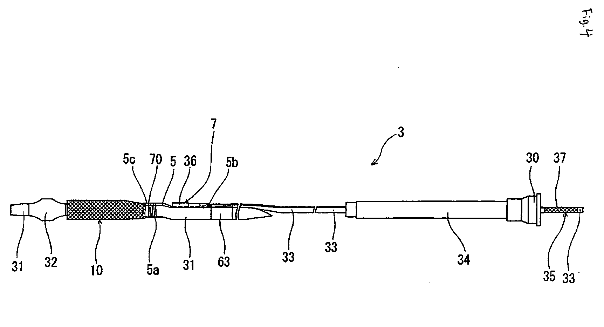

   FIG. 4 is a partly omitted front view of a shaft section in the stent delivery system shown in FIG. 1.
[FIG. 5]

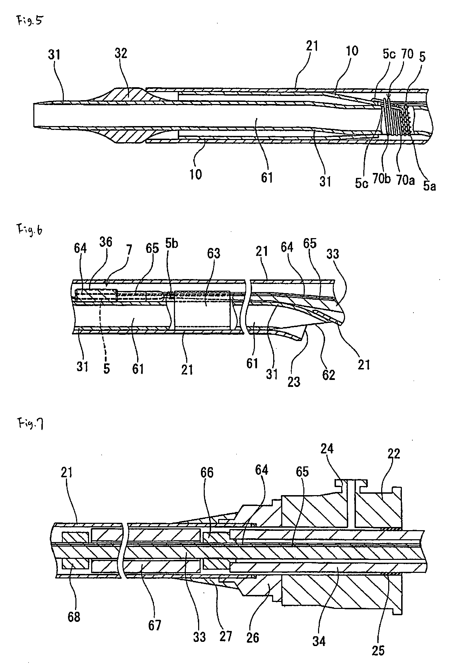

   FIG. 5 is an enlarged longitudinal sectional view of the vicinity of a distal portion in the stent delivery system shown in FIG. 1.
[FIG. 6]
   FIG. 6 is an enlarged longitudinal sectional view of the vicinity of an intermediate portion in the stent delivery system shown in FIG. 1.
[FIG. 7]
   FIG. 7 is an enlarged longitudinal sectional view of the vicinity of a proximal portion of the sheath in the stent delivery system shown in FIG. 1.
[FIG. 8]

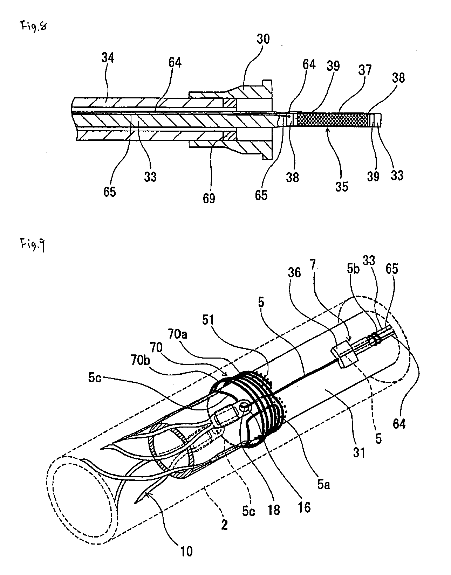

   FIG. 8 is an enlarged longitudinal sectional view of the vicinity of a proximal portion of the shaft in the stent delivery system shown in FIG. 1.
[FIG. 9]
   FIG. 9 is an illustration for describing the vicinity of a proximal portion of a stent in the stent delivery system shown in FIG. 1.
[FIG. 10]

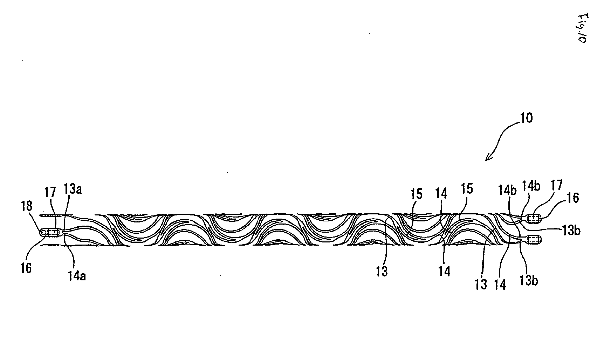

   FIG. 10 is a front view of an example of a stent for placement in living body which is used in the stent delivery system according to the present invention.
[FIG. 11]

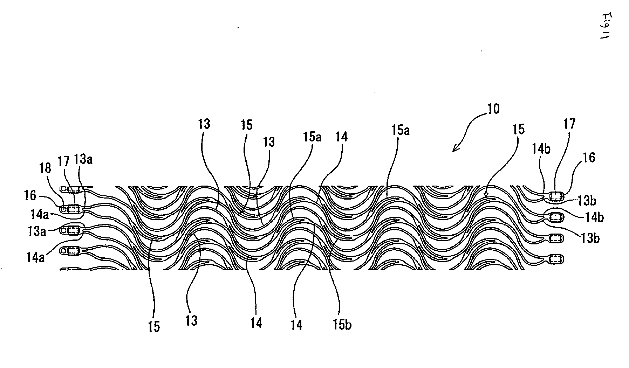

   FIG. 11 is a development of the stent for placement in living body of FIG. 10.
[FIG. 12]

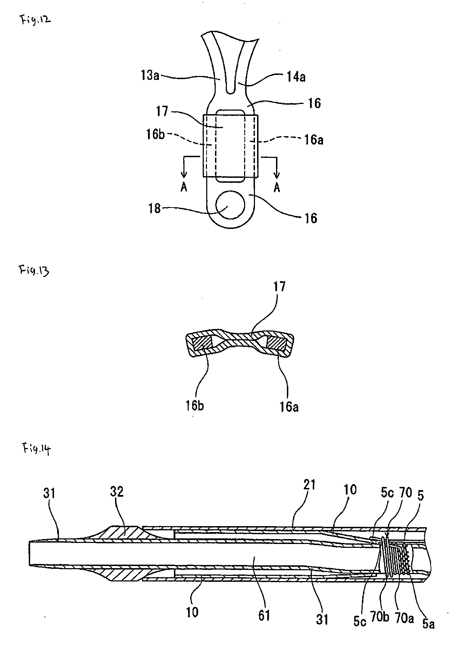

   FIG. 12 is an enlarged view of the vicinity of a small hole in a proximal portion of the stent shown in FIG. 10.
[FIG. 13]
   FIG. 13 is an enlarged sectional view taken along line A-A of FIG. 12.
[FIG. 14]
   FIG. 14 is an enlarged longitudinal sectional view of the vicinity of a distal portion in the stent delivery system according to another embodiment of the present invention.
[FIG. 15]

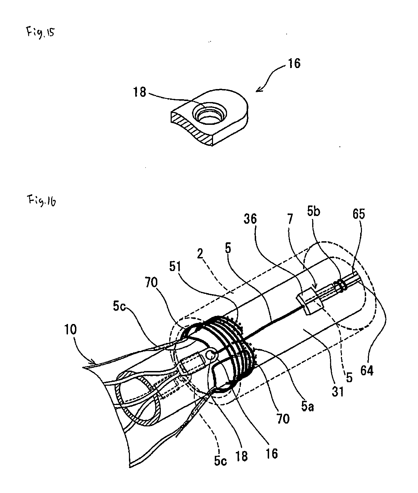

   FIG. 15 is an enlarged perspective view of the vicinity of a small hole in a proximal portion of the stent used in the stent delivery system according to the present invention.
[FIG. 16]
   FIG. 16 is an illustration for describing the operation of the stent delivery system according to the present invention.
[FIG. 17]

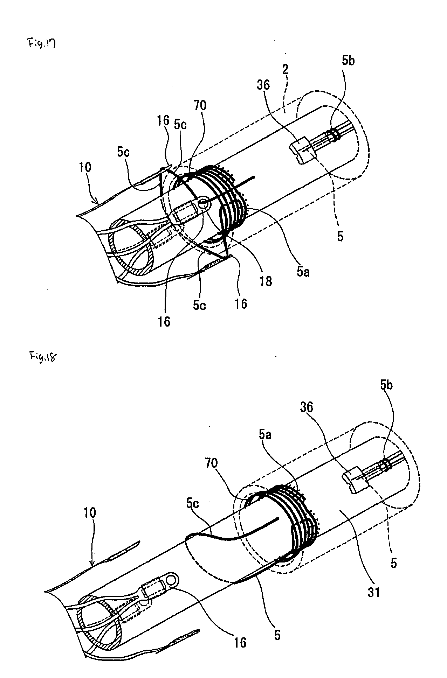

   FIG. 17 is an illustration for describing the operation of the stent delivery system according to the present invention.
[FIG. 18]
   FIG. 18 is an illustration for describing the operation of the stent delivery system according to the present invention.

### Best Mode for Carrying Out the Invention

A stent delivery system according to the present invention will be described referring to the following preferred embodiments. The stent delivery system is, in other words, a living organ dilator.

The stent delivery system 1 according to the present invention includes: a stent 10 which is formed to be substantially cylindrical in shape, is compressed toward its center axis at the time of insertion into a living body, and is capable of expanding outward when placed (put indwelling) in the living body, to be restored into its pre-compression shape; a shaft section 3 having a guide wire lumen 61; and a sheath 2 having the stent 10 contained in a distal portion thereof, with the stent 10 being located at a position which is on the shaft section 3 and near the distal end of the shaft section 3. Further, the stent delivery system 1 includes a stent proximal portion fixing wire 5 having one end portion 5a and the other end portion 5b fixed to the shaft section 3 and having an intermediate portion 5c engaged with a proximal portion of the stent 10, and a breaking member 7 for breaking the stent proximal portion fixing wire 5 to release the stent from the engagement.
In addition, the stent delivery system 1 in the embodiment shown in the drawings includes the stent 10 capable of expanding outward when placed (put indwelling) in a living body, to be restored into its pre-compression shape, the sheath 2 having the stent 1 contained in a distal portion thereof, and the shaft section 3 which is slidably inserted in the sheath 2 and which is for discharging the stent 10 through the distal end of the sheath 2. The stent 10 used here has a distal portion oriented toward the distal side of the sheath 2 and a proximal portion oriented toward the proximal side of the sheath 2. Further, the stent 10 substantially does not have any bent free end at least projecting toward the proximal side, exclusively of the proximal portion. In addition, by moving the sheath 2 after exposure of a distal portion of the stent 10 from the sheath 2, the exposed distal portion can be re-contained into the sheath 2. The stent delivery system 1 has the guide wire lumen 61 which has one end opening at the distal end of the stent delivery system, and the other end opening on the proximal side relative to a stent-containing part of the sheath 2. The shaft section 3 includes the stent proximal portion fixing wire 5 having one end portion 5a and the other end portion 5b fixed to the shaft section 3 and having an intermediate portion 5c engaged with a proximal portion of the stent 10, and the breaking member 7 for breaking the stent proximal portion fixing wire 5 to release the stent 10 from the engagement.

The stent delivery system 1 according to the present invention is composed of the stent 10, the sheath 2 having the stent 10 contained in a distal portion thereof, and the shaft section 3 slidably inserted in the sheath 2.
As shown in FIGS. 1 to 7, the sheath 2 includes a sheath tube 21, and a sheath hub 22 fixed to the proximal end of the sheath tube 21.
As shown in FIGS. 1 to 7, the sheath tube 21 is a tubular body, which is opened at its front end (tip) and its rear end. The tip opening functions as a discharge port for the stent 10 when the stent 10 is placed (put indwelling) in a stenosed lesion in a body lumen. By being pushed out via the tip opening, the stent 10 is released from a stress load, to expand and to be restored into its pre-compression shape. The distal portion of the sheath tube 21 constitutes a stent-containing part 21a for containing the stent 10 therein. In addition, the sheath tube 21 has a side hole 23 provided on the proximal side relative to the stent-containing part 21a. The side hole 23 is for leading out a guide wire to the exterior.
The outside diameter of the sheath tube 21 is preferably about 0.5 to 4.0 mm, particularly 0.8 to 2.0 mm. The inside diameter of the sheath tube 21 is preferably about 0.2 to 1.8 mm. The length of the sheath tube 21 is preferably 300 to 2500 mm, particularly about 300 to 2000 mm.
The material for forming the sheath tube 21 is appropriately selected while taking the physical properties required of the sheath tube (flexibility, hardness, strength, slidability, anti-kinking property, and expansion/contraction properties) into consideration. Preferably, the material is selected, for example, from among polyethylene, polypropylene, nylon, polyethylene terephthalate, fluoro-polymers such as PTFE, ETFE, etc. and thermoplastic elastomers. The thermoplastic elastomers are appropriately selected from among nylon-based ones (e.g., polyamide elastomer), urethane-based ones (e.g., polyurethane elastomer), polyester-based ones (e.g., polyethylene terephthalate elastomer), and olefin-based ones (e.g., polyethylene elastomer, polypropylene elastomer).

Further, an outer surface of the sheath tube 21 is preferably subjected to a treatment for rendering the outer surface lubricious. Examples of such a treatment include coating the outer surface with a hydrophilic polymer or fixing a hydrophilic polymer to the outer surface, wherein examples of the hydrophilic polymer include poly(2-hydroxyethyl methacrylate), polyhydroxyethyl acrylate, hydroxypropyl cellulose, methyl vinyl ether-maleic anhydride copolymer, polyethylene glycol, polyacrylamide, polyvinyl pyrrolidone, etc. In addition, an inner surface of the sheath tube 21 may be coated with the above-mentioned hydrophilic polymer or the hydrophilic polymer may be fixed to the inner surface, in order to enhance slidability between the inner surface and the stent 10 as well as the shaft section 3.
In addition, as shown in FIGS. 1 to 3 and 7, a sheath hub 22 is fixed to a proximal portion of the sheath tube 21. As shown in FIG. 7, the sheath hub 22 has a seal member 25 which holds the shaft section 3 in a slidable and liquid-tight manner. Besides, the sheath hub 22 has a side port 24.
As the material for forming the sheath hub 22, a hard or semi-hard material is used. Examples of the hard or semi-hard material which can be used here include synthetic resins such as polycarbonate, polyolefins (e.g., polyethylene, polypropylene, ethylene-propylene copolymer), styrene reins [e.g., polystyrene, MS resin (methacrylate-styrene copolymer), MBS resin (methacrylate-butylene-styrene copolymer)], polyesters, etc., and metal such as stainless steel, aluminum, aluminum alloys, etc.
Besides, as the material for forming the seal member 25 and an elastic ring 69 to be described later, an elastic material is used. Examples of the elastic material include rubbers such as synthetic rubbers such as urethane rubber, silicone rubber, butadiene rubber, etc. and natural rubbers such as latex rubber, etc., and synthetic resin elastomers such as olefin elastomers (e.g., polyethylene elastomer, polypropylene elastomer), polyamide elastomers, styrene elastomers (e.g., styrenebutadiene-styrene copolymer, styrene-isoprene-styrene copolymer, styrene-ethylenebutylene-styrene copolymer), polyurethane, urethane elastomers, fluoro-resin elastomers, etc.
In addition, at a distal portion of the sheath hub 22, there are provided reinforcement members 26, 27 which extend toward the distal side from the distal end of the sheath hub.

As shown in FIGS. 1 to 8, the shaft section 3 includes: a shaft body 33; a distal tube 31 provided at the distal end of the shaft body 33 and protruding from the distal end of the sheath 2; a shaft hub 30 fixed to a proximal portion of the shaft body 33; a stent proximal portion fixing wire 5 fixed to the shaft body 33; and a breaking member 7 provided on the shaft body 33 for the purpose of breaking the stent proximal portion fixing wire 5.
In this embodiment, the stent proximal portion fixing wire 5 is a heat-breaking stent proximal portion fixing wire, and the breaking member 7 is a heat-breaking member. Incidentally, this configuration is not limitative; the stent proximal portion fixing wire 5 and the breaking member 7 may be ones for breaking the stent electrically, mechanically, or by water pressure or the like, thereby being released from the shaft section 3.
Besides, in this embodiment, the shaft section 3 has a proximal-side opening of the guide wire lumen opening at a side part on the proximal side relative to the stent-containing part of the sheath 2, whereas the sheath 2 has a sheath side hole provided on the proximal side relative to the stent-containing part, and a guide wire can be inserted through the sheath side hole and the proximal-side opening.

As shown in FIG. 5, the distal tube 31 protrudes from the distal end of the sheath 2. In addition, the distal tube 31 is provided with a stopper 32 for inhibiting the sheath 2 from moving in the distal direction. As shown in FIG. 6, a proximal portion of the distal tube 31 is curved, is entering into a side hole 23 of the sheath tube 21, and is disengageably engaged with the side hole 23. The outside diameter of the distal tube 31 is preferably 0.2 to 1.8 mm. Besides, a distal portion of the distal-side stopper 32 is preferably reduced in diameter toward the distal direction, as shown in FIG. 5. The outside diameter of a maximum-diameter portion of the stopper 32 is preferably 0.5 to 4.0 mm. In addition, a proximal portion of the stopper 32 is also preferably reduced in diameter toward the proximal direction, as shown in FIG. 5. Besides, the distal tube 31 has the guide wire lumen 61 extending from the distal end to the proximal end thereof. The position of a proximal opening 62 of the guide wire lumen 61 is preferably deviated to the proximal side from the distal end of the distal tube 31 by 10 to 400 mm, particularly 50 to 350 mm. In addition, the position of the proximal opening 62 is preferably deviated to the proximal side from the rear end of the stent 10 arranged (in other words, the rear end of the stent-containing part) by about 50 to 250 mm.
The shaft body 33 has a distal section fixed to a proximal portion of the distal tube 31, a body section extending proximally over a predetermined distance, and a proximal section protruding from the shaft hub 30. In this embodiment, the shaft body 33 has a structure in which a distal portion of its part fixed to the distal tube 31 is a small-diameter section, and the body section and the proximal section are greater than the small-diameter section in outside diameter. In this embodiment, the distal section of the shaft body 33 is fixed to a side surface of the distal tube 31 through a heat-shrinking tube 63.
The length of the shaft section 3 is preferably about 400 to 2500 mm, particularly 400 to 2200 mm. In addition, the outside diameter of the body section of the shaft body 33 is preferably about 1.0 to 2.5 mm, particularly 1.0 to 2.0 mm. Besides, the length of the distal tube 31 is preferably about 10 to 400 mm, particularly 50 to 350 mm, and the outside diameter of the distal tube 31 is preferably about 0.2 to 2.0 mm. In addition, the inside diameter of the lumen 61 is preferably about 0.2 to 2.0 mm, particularly 0.3 to 1.0 mm.

The shaft body 33 may be either solid or tubular. In addition, it may be a coil shaft. The material for forming the shaft section 3 is preferably a material which has hardness and a certain degree of flexibility. Preferable examples of the material include metallic wires or metallic pipes of stainless steel, superelastic metals and the like, and bar-like bodies or annular bodies or the like of polyethylene, polypropylene, nylon, polyethylene terephthalate, fluoro-polymers such as ETFE, etc., PEEK (polyether ether ketone), polyimide, etc. Incidentally, an outer surface of the shaft section 3 may be coated with a resin having bio-compatibility, particularly, anti-thrombotic property. Examples of the anti-thrombotic material which can be preferably used here include polyhydroxyethyl methacrylate, copolymers of hydroxyethyl methacrylate and styrene (e.g., HEMA-St-HEMA block copolymer), etc.
Further, the outer surface of that portion of the shaft section 3 which may protrude from the sheath 2, preferably, has lubricity. In view of this, the outer surface may be coated with a hydrophilic polymer or a hydrophilic polymer may be fixed to the outer surface, wherein examples of the hydrophilic polymer include poly(2-hydroxyethyl methacrylate), polyhydroxyethyl acrylate, hydroxypropyl cellulose, methyl vinyl ether-maleic anhydride copolymer, polyethylene glycol, polyacrylamide, polyvinyl pyrrolidone, etc. In addition, the whole part of the outer surface of the shaft section 3 may be coated with the above-mentioned hydrophilic polymer or the hydrophilic polymer may be fixed to the whole part of the outer surface. Furthermore, an inner surface of the shaft section 3 may be coated with the above-mentioned hydrophilic polymer or the hydrophilic polymer may be fixed to the inner surface, in order to enhance slidability between the inner surface and a guide wire.
The shaft body 33 penetrates the sheath 2, and protrudes from the rear-end opening of the sheath 2. As shown in FIGS. 1 to 3 and 8, the shaft hub 30 is firmly attached to a proximal portion of the shaft body 33. In this embodiment, a fixation ring 66 is fixed to the shaft body 33, as shown in FIG. 7. In addition, a proximal tube 34 which extends toward the distal side from the shaft hub 30 is fixed to the shaft hub 30. A distal portion of the proximal tube 34 is fixed to the fixation ring 66. Besides, an elastic ring 69 is fixed to the proximal end of the proximal tube 34 (in the inside of the shaft hub 30). Further, in this embodiment, a second fixation ring 68 is provided at a predetermined distance to the distal side relative to the fixation ring 66. In addition, an intermediate tube 67 is disposed between the fixation ring 66 and the second fixation ring 68. The intermediate tube 67 is fixed to neither the shaft body 33 nor the sheath tube 21, and can abut on both the fixation ring 66 and the second fixation ring 68. The arrangement of such an intermediate tube promises favorable sliding of the sheath. The intermediate tube 67 is preferably a tube which has a low-friction surface. Specifically, a tube formed of, for example, polyethylene, polypropylene, nylon, polyethylene terephthalate, a fluoro-polymers such as PTFE and ETFE, or the like is preferably used.

Furthermore, as shown in FIGS. 5 and 9, a proximal-side stopper 70 for restraining the stent 10 from moving in the proximal direction is provided at a distal portion of the shaft section 3 (specifically, near the proximal end of that part of the distal tube 31 at which the stent is disposed). Particularly, in this embodiment, the proximal-side stopper 70 is a spring-formed stopper wound around the shaft section. As shown in FIGS. 5 and 9, the proximal-side stopper 70 includes a proximal-side coil section 70a wound around the distal tube 31, and a distal-side coil section 70b which extends to the distal side from the proximal-side coil section 70a and which has a part not in contact with the distal tube 31. The distal-side coil section 70b in this embodiment is eccentrically fixed to the distal tube 31, and has both a part in contact with the distal tube 31 and a part separate from the distal tube 31. In addition, the stent proximal portion fixing wire 5 penetrates that part of the distal-side coil section 70b which is not in contact with the distal tube 31. Besides, the wire 5 at a part extending in the direction of the stent from one end part 5a of the wire may be fixed to the above-mentioned distal-side coil section 70b. The fixation of the wire 5 to the distal-side coil section 70b is preferably achieved by clamping between the turns of the coil. In addition, the distal-side coil section 70b functions as a stopper for the stent 10. Further, the distal-side coil section 70b may be so configured that substantially the whole part thereof is separate from the distal tube 31, as in the embodiment shown in FIG. 14.

Besides, the proximal-side coil section 70a is spring-formed, and can lock a proximal portion of the stent without damaging the latter. In addition, the stopper 70 may be formed of a radiopaque material. This ensures that the position of the vicinity of the proximal end of the stent can be grasped under radioscopy, and the procedure is facilitated. Preferable examples of the radiopaque material include gold, platinum, platinum-iridium alloy, silver, stainless steel, platinum, their alloys, etc. The stopper 70 is formed by forming a wire from the radiopaque material and winding the wire around the outer surface of the distal tube 31.
Further, as shown in FIGS. 4 to 6 and 9, the shaft section 3 includes the heat-breaking stent proximal portion fixing wire 5 having one end portion 5a and the other end portion 5b fixed to the shaft section and having the intermediate portion 5c engaged with a proximal portion of the stent 10, and the heat-breaking member 7 for breaking the stent proximal portion fixing wire 5 to release the stent 10 from the engagement.
Particularly, in this embodiment, as shown in FIG. 9, the stent 10 used is a stent which has a plurality of small holes 18 for permitting the stent proximal portion fixing wire to pass therethrough, the small holes 18 being provided in proximal-side joint sections 16 in a substantially annular pattern. Further, the intermediate portion 5c of the stent proximal portion fixing wire 5 penetrates sequentially the plurality of small holes 18 of the stent 10, and passes through the plurality of small holes 18 in an annular pattern as a whole. Therefore, the stent 10 is engaged with (fixed to) the shaft section 3 by the stent proximal portion fixing wire 5, and would not be released from the shaft section 3 unless the stent proximal portion fixing wire 5 is broken (cut).

Particularly, in this embodiment, one end portion 5a of the stent proximal portion fixing wire 5 is wound around the outer surface of the distal tube 31 and fixed there by an adhesive 51, in the vicinity of and slightly on the proximal side of the stopper 70. In addition, the other end portion 5b of the stent proximal portion fixing wire 5 is wound around the outer surface of the shaft body 33 and is fixed there. Incidentally, one end portion 5a and the other end portion 5b of the stent proximal portion fixing wire 5 are not limited to those which are wound around and fixed to the outer surfaces of the distal tube 31 and the shaft body 33, respectively. One end portion 5a and the other end portion 5b of the stent proximal portion fixing wire 5 may be fixed respectively to the outer surfaces of the distal tube 31 and the shaft body 33 by caulking rings. Further, in this embodiment, the stent proximal portion fixing wire 5 extends in the direction of the stent by passing through the gaps in the coil constituting the spring-formed stopper 70, from one end portion 5a and the other end portion 5b which are fixed to the shaft section. Specifically, the stent proximal portion fixing wire 5 at the part extending from one end portion 5a and the stent proximal portion fixing wire 5 at the part extending from the other end portion 5b are both extending by passing over the proximal-side coil section 70a of the stopper 70 and penetrating between the distal-side coil section 70b and the distal tube 31. With the stopper formed in this manner, it exhibits an effect as a stopper for the stent proximal portion, and guides (penetrates) the stent fixing wire, whereby fixation of the stent by the wire is made assured. In addition, at the time of releasing the wire from the stent, the wire is prevented from be entangled on the stent, so that the release can be performed assuredly.

The heat-breaking stent proximal portion fixing wire 5 is preferably a thermoplastic resin fiber. The thermoplastic resin is preferably such a synthetic resin as polyethylene, polypropylene, nylon, polyethylene terephthalate, etc., particularly, one that has a low melting point. The heat-breaking stent proximal portion fixing wire may have a configuration in which only its portion near the part to be heat-broken is formed of a low-melting-point resin. Besides, the heat-breaking stent proximal portion fixing wire may be composed of a single thermoplastic resin fiber, a plurality of thermoplastic resin fibers bundled together or twisted together, or the like. The shaft section 3 has the heat-breaking member 7 for breaking the stent proximal portion fixing wire 5 to release the stent 10 from the engagement. In this embodiment, the heat-breaking member 7 includes a heat generating section 36 for breaking, electric cables 64, 65 having distal portions connected to the heat generating section and extending to a proximal portion of the shaft body 33, and a joint section 35 for joint to a power supply device, the joint section 35 being formed at a proximal portion of the shaft body 33. Particularly, in this embodiment, the heat generating section 36 for breaking of the heat-breaking member 7 is fixed to the distal end of the shaft body 33, and the electric cables 64, 65 extend to the proximal portion of the shaft body 33 while being fixed to the outer surface of the shaft body 33.
The joint section 35 for joint to the power supply device (not shown) is formed at the proximal portion of the shaft body 33. The joint section 35 is formed at the outer surface of the proximal potion of the shaft body 33, and includes a first electrode part 37 electrically connected to the cable 64, and a second electrode 38 connected to the cable 65. Besides, in this embodiment, an insulating part 39 is provided for insulation between the first electrode 37 and the second electrode 38. A part of the heat-breaking stent proximal portion fixing wire 5, in this embodiment, a part deviated by a predetermined distance toward an intermediate part side relative to the other end portion 5b, is enveloped by the heat generating section 36 for breaking. By electric power supplied to the first electrode part 37 and the second electrode 38 of the joint section 35, the heat generating section 36 for breaking generates heat, whereby the heat-breaking stent proximal portion fixing wire 5 is broken through fusion at the position of the heat generating section 36.

The stent 10 to be used in the present invention is a so-called self-expandable stent which is capable of expanding outward when placed (put indwelling) in a living body, to be restored into its pre-compression shape. Further, the stent 10 includes a distal portion oriented toward the distal side of the sheath 2 and a proximal portion oriented toward the proximal side of the sheath 2. Furthermore, the stent 10 substantially does not have any bent free end at least projecting toward the proximal side, exclusively of the proximal portion thereof. By moving the sheath 2 after exposure of a distal portion of the stent 10 from the sheath 2, the exposed distal portion can be re-contained into the sheath 2.
The stent to be used may be a stent such that an apex of a proximal-side bent portion or a part near the apex is joined to another linear element, so that the stent does not have a free end. Besides, the stent to be used may be a stent as shown in FIGS. 10 and 11. FIG. 10 is a front view of an example of a stent for placement in living body which is used in the stent delivery system according to the present invention. FIG. 11 is a development of the stent for placement in living body of FIG. 10.
This stent 10 includes pluralities of wave-shaped struts 13 and 14 extending in the axial direction of the stent from one end side to the other end side of the stent and arranged in the circumferential direction of the stent, and one or more connecting struts 15 for each interconnecting the wave-shaped struts which are adjacent to each other, the one or more connecting struts 15 extending in the axial direction over a predetermined length. Further, each of end portions of each of the wave-shaped struts 13, 14 is joined to an end portion of a wave-shaped strut close to the each wave-shaped strut of concern.

Particularly, the stent 10 shown in FIGS. 10 and 11 includes: a plurality of first wave-shaped struts 13 extending in the axial direction of the stent 10 from one end side to the other end side of the stent 10 and arranged in the circumferential direction of the stent; a plurality of second wave-shaped struts 14 located between the first wave-shaped struts 13, extending in the axial direction of the stent from one end side to the other end side of the stent, and arranged in the circumferential direction of the stent; one or more connecting struts 15 for each interconnecting the first wave-shaped strut 13 and the second wave-shaped strut 14 which are adjacent to each other, the one or more connecting struts 15 extending in the axial direction over predetermined length. Apexes of the second wave-shaped struts 14 are shifted a predetermined distance in the axial direction of the stent relative to those apexes of the first wave-shaped struts 13 which are close to the apexes of the second wave-shaped struts 14 in the circumferential direction of the stent 10 and are curved in the same direction as the apexes of the second wave-shaped struts 14. In addition, the first wave-shaped strut 13 has end portions 13a, 13b joined to end portions 14a, 14b of the second wave-shaped strut close thereto.
The stent 10 in this embodiment is a so-called self-expandable stent which is formed in a substantially cylindrical shape, is compressed toward its center axis at the time of insertion into a living body, and expands outward when placed (put indwelling) in the living body, to be restored into its pre-compression shape.

The first wave-shaped struts 13 extend in the axial direction substantially in parallel to the center axis of the stent. A plurality of the first wave-shaped struts 13 are arranged in the circumferential direction of the stent. The number of the first wave-shaped struts 13 is preferably three or more, particularly about three to eight. Further, the plurality of the first wave-shaped struts 13 are preferably so arranged that they are substantially at an equal angle to the center axis of the stent.
The second wave-shaped struts 14 also extend in the axial direction substantially in parallel to the center axis of the stent. A plurality of the second wave-shaped struts 14 are arranged in the circumferential direction of the stent, and each of the second wave-shaped struts 14 is disposed between the first wave-shaped struts. The number of the second wave-shaped struts 14 is preferably three or more, particularly about three to eight. Further, the plurality of the second wave-shaped struts 14 are preferably so arranged that they are substantially at an equal angle to the center axis of the stent. In addition, the number of the second wave-shaped struts 14 is the same as the number of the first wave-shaped struts 13.
The stent 10 has one or more connecting struts 15 each of which interconnects the first wave-shaped strut 13 and the second wave-shaped strut 14 adjacent to each other and which extend in the axial direction over a predetermined length. Particularly, in the stent 10 in this embodiment, the connecting strut 15 has one end near an inflection point of the wave-shaped strut on one side, has the other end in a region ranging from a position near an apex of that wave-shaped strut on the other side which is adjacent to the wave-shaped strut on one side to a position slightly beyond the apex, extends in the axial direction, and is curved in the same direction as an apex of the wave-shaped strut on the other side. Specifically, as shown in FIG. 11, the connecting struts 15 are composed of first connecting struts 15a which have apexes directed to one side in the circumferential direction of the stent 10 and are curved, and second connecting struts 15b which have apexes directed to the other side in the circumferential direction of the stent 10 and are curved. Besides, the connecting strut 15 is curved in a circular arc shape, and has a radius approximately equal to the radius of the circular arc shape of a curved part of the first wave-shaped strut 13 or second wave-shaped strut 14 close thereto in the circumferential direction of the stent 10.

Besides, the stent 10 in this embodiment has the joint sections 16 by which every one of one-end-side end portions and the-other-end-side end portions of all the first wave-shaped struts is joined to an end portion of either one of the second wave-shaped struts close to the first wave-shaped strut. Specifically, one-end-side end portion 13a of the first wave-shaped strut of the stent 10 is joined to one-side end portion 14a of that second wave-shaped strut 14 on one side which is close to the first wave-shaped strut (specifically, that second wave-shaped strut 14 which is located close to and on the other side in the circumferential direction of the first wave-shaped strut) by the joint section 16. In addition, the-other-end-side end portion 13b of the first wave-shaped strut is joined to the-other-end-side end portion 14b of that second wave-shaped strut 14 on one side which is close to the first wave-shaped strut (specifically, that second wave-shaped strut 14 which is located close to and on one side in the circumferential direction of the first wave-shaped strut) by the joint section 16. In other words, the joint section 16 on one end side and the joint section 16 on the other end side are different from each other (shifted one at a time) in combination of the first wave-shaped strut 13 and the second wave-shaped strut 14 which are joined to each other.
The joint sections 16 are each fitted with a radiopaque marker 17, as shown in FIGS. 10 to 12. In this embodiment, as shown in FIG. 12, the joint section 16 has two frame parts 16a, 16b extending toward the end portion in parallel to each other with a predetermined spacing therebetween, and the radiopaque marker 17 envelopes the two frame parts 16a, 16b substantially entirely or partly. In addition, the radiopaque marker 17 is in the shape of a thin-walled rectangular parallelepiped, accommodates the two frame parts 16a, 16b therein, and is recessed in a central area so as to be fixed to the two frame parts 16a, 16b. Examples of the material which can be suitably used to form the radiopaque marker include one (elemental substance) or two or more (alloy) selected from the group consisting of iridium, platinum, gold, rhenium, tungsten, palladium, rhodium, tantalum, silver, ruthenium, and hafnium.
Further, the stent 10 is provided, in each of the joint sections 16 on the proximal side portion, with a small hole 18 for permitting the stent proximal portion fixing wire to pass therethrough. The small holes 18 extend in the direction of the center of the stent. The small hole 18 for permitting the stent proximal portion fixing wire to pass therethrough, preferably, has a low-friction inner surface or an easily releasable form for enhancing releasability of the wire 5 therefrom. The low-friction inner surface can be formed by making the inner surface a smooth surface, or coating the inner surface with a low-friction material, or the like method.

In addition, as the easily releasable form of the small holes, there can be considered the form as shown in FIG. 15. The small hole 18 formed in the joint section 16 shown in FIG. 15 has a form in which the edge of the opening of the small hole 18 is chamfered or enlarged in diameter in a tapered form. Incidentally, the small hole 18 may be chamfered or enlarged in diameter in a tapered form, at both the edges of the opening on the outer surface side and the inner surface side of the stent. This promises easy passage and easy release of the stent fixing wire.
The material constituting the stent 10 is preferably a superelastic metal. As the superelastic metal, superelastic alloys are preferably used. The superelastic alloys here means those alloys which are generally called shape memory alloys and which show superelasticity at least at a living body temperature (around 37°C). Particularly preferable for use here are superelastic metal bodies such as Ti-Ni alloys containing 49 to 53 atomic% of Ni, Cu-Zn alloys containing 38.5 to 41.5 wt% of Zn, Cu-Zn-X alloys (X = Be, Si, Sn, Al, Ga) containing 1 to 10 wt% of X, Ni-Al alloys containing 36 to 38 atomic% of Al, etc. Especially preferred are the above-mentioned Ti-Ni alloys. Besides, use of Ti-Ni-X alloys (X = Co, Fe, Mn, Cr, V, Al, Nb, W, B or the like) prepared by replacing part of the Ti-Ni alloys by 0.01 to 10.0% of X, use of Ti-Ni-X alloys (X = Cu, Pb, Zr) prepared by replacing part of the Ti-Ni alloys by 0.01 to 30.0% of atoms, or selection of cold working ratio and/or final heat treatment conditions, may be made, whereby mechanical properties of the superelastic alloy can be changed, as required. Besides, while using the above-mentioned Ti-Ni-X alloy, the cold working ratio and/or final heat treatment conditions may be selected, whereby the mechanical properties of the alloy can be changed, as required. Of the superelastic alloy to be used, the buckling strength (the yield stress under load) is 5 to 200 kg/mm² (22°C), preferably 8 to 150 kg/mm², and the restoring stress (the yield stress when unloaded) is 3 to 180 kg/mm² (22°C), preferably 5 to 130 kg/mm². The superelasticity here means a property of a metal such that even upon deformation (bending, extension, compression) of the metal into a region where ordinary metals undergo plastic deformation at use temperature, the deformed metal is restored substantially into its pre-compression shape after release of the deformation, without needing heating.
The diameter of the stent in a compressed state is preferably about 0.5 to 1.8 mm, particularly 0.6 to 1.4 mm. In addition, the length of the stent in a non-compressed state is preferably about 5 to 200 mm, particularly 8.0 to 100.0 mm. In addition, the diameter of the stent in a non-compressed state is preferably about 1.5 to 6.0 mm, more preferably 2.0 to 5.0 mm. Further, the material thickness of the stent is preferably about 0.05 to 0.40 mm, particularly 0.05 to 0.15 mm. The width of the wave-shaped struts is preferably 0.01 to 1.00 mm, particularly 0.05 to 0.2 mm. Surfaces of the wave-shaped struts are preferably in the state of having been processed to be smooth; in this case, smoothening is preferably carried out by electropolishing. Besides, the strength of the stent in the radial direction is preferably 0.1 to 30.0 N/cm, particularly 0.5 to 5.0 N/cm.

Now, operation of the stent delivery system according to the present invention will be described below, referring to FIGS. 9 and 16 to 18.
When the whole body of the stent 10 is contained in the sheath 2, the condition is as shown in FIG. 9. Then, with the sheath 2 slid toward the proximal side, as shown in FIG. 16, the stent 10 is exposed from the distal opening of the sheath 2. The stent 10 exposed from the sheath 2 shows tendency to expand by its self-expanding force so as to be restored into its pre-compression state. In this stent delivery system, however, the proximal portion of the stent 10 is engaged with the shaft section 3 by the heat-breaking stent proximal portion fixing wire 5, so that the stent 10 cannot expand, and stays in the state shown in FIG. 16. In the case where it is necessary to re-adjust the arrangement position of the stent 10, the stent 10 can be re-contained into the sheath by sliding the sheath 2 in the distal direction. After it is confirmed that the stent 10 is disposed in a target lesion, the power supply device (not shown) connected to the shaft section 3 is operated to cause the heat generating section 36 for breaking to generate heat, thereby braking the stent proximal portion fixing wire 5. As a result, the proximal portion of the stent 10 is released from the engagement made by the heat-breaking stent proximal portion fixing wire 5, and the proximal portion also expands, as shown in FIG. 17. Thereafter, the stent delivery system 1 (the sheath 2 and the shaft section 3) having the stent released therefrom is moved in the proximal direction, whereby the intermediate portion 5c of the stent proximal portion fixing wire 5 having held the stent 10 in engagement is released from the stent, as shown in FIG. 18. Incidentally, the broken stent proximal portion fixing wire 5 inclusive of the intermediate portion 5c has one end fixed to the shaft section 3, and, therefore, the wire 5 is neither discharged into the living body nor left remaining in the stent.

### Industrial Applicability

The stent delivery system according to the present invention is as follows.
(1) A stent delivery system including: a stent which is formed to be substantially cylindrical in shape, is compressed toward a center axis thereof at the time of insertion into a living body, and is capable of expanding outward when placed in the living body, to be restored into a pre-compression shape thereof; a shaft section having a guide wire lumen; and a sheath having the stent contained in a distal portion thereof, the stent being located at a position which is on the shaft section and near the distal end of the shaft section; wherein the stent delivery system includes a stent proximal portion fixing wire having one end portion and the other end portion fixed to the shaft section, and having an intermediate portion engaged with a proximal portion of the stent, and a breaking member for breaking the stent proximal portion fixing wire to release the stent from the engagement.
This ensures that the proximal portion of the stent is in engagement with the shaft section until the stent proximal portion fixing wire is broken, and, therefore, the stent would not jump out at the time of discharge from the sheath. In addition, even after discharge of the stent from the sheath, the stent can be re-contained into the sheath if the stent proximal portion fixing wire is not yet broken. Accordingly, the arrangement position of the stent can be corrected, and the stent can be assuredly disposed in a target site.
Further, if the stent proximal portion fixing wire extends in the direction of the stent by passing through the gap in the coil constituting the spring-formed stopper from one end portion and the other end portion fixed to the shaft section, the position of the wire is stabilized; therefore, fixation of the stent by the wire is assured, and the wire can be released from the stent favorably.

Embodiments of examples may be as follows.
(2) The stent delivery system as described in paragraph (1) above, wherein the stent is provided in a proximal portion thereof with a plurality of small holes permitting the stent proximal portion fixing wire to pass therethrough, the small holes being provided in a substantially annular pattern, and the intermediate portion of the stent proximal portion fixing wire is passed annularly through the plurality of small holes in the stent.
(3) The stent delivery system as described in paragraph (1) above, wherein the stent has a plurality of proximal direction bent portions located at a proximal portion thereof, and the intermediate portion of the stent proximal portion fixing wire is passed annularly through the plurality of proximal direction bent portions of the stent.
(4) The stent delivery system as described in any of paragraphs (1) to (3) above, wherein the shaft section has a distal tube having the guide wire lumen, and a shaft body having a distal portion fixed to the proximal side of the distal tube, and the breaking member is provided at the distal portion of the shaft body.
(5) The stent delivery system as described in any of paragraphs (1) to (4) above, wherein the stent has a distal portion oriented toward the distal side of the sheath and a proximal portion oriented toward the proximal side of the sheath; the stent substantially does not have any bent free end at least projecting toward the proximal side, exclusively of the proximal portion; and, after exposure of the distal portion from the sheath, the exposed distal portion can be re-contained into the sheath by moving the sheath.

(6) The stent delivery system as described in any of paragraphs (1) to (5), wherein the stent proximal portion fixing wire is a heat-breaking stent proximal portion fixing wire, and the breaking member is a heat-breaking member.
(7) The stent delivery system as described in paragraph (6) above, wherein the heat-breaking member includes a heat generating section for breaking, an electric cable having a distal portion connected to the heat generating section and extending to a proximal portion of the shaft body, and a joint section for joint to a power supply section, the joint section being connected to the electric cable and formed at the proximal portion of the shaft body.
(8) The stent delivery system as described in any of paragraphs (1) to (7) above, wherein the shaft section has a proximal-side opening of the guide wire lumen, the proximal-side opening opened at a side portion on the proximal side relative to a stent-containing part of the sheath; the sheath has a sheath side hole provided on the proximal side relative to the stent-containing part; and the guide wire can be inserted through the sheath side hole and the proximal-side opening.
(9) The stent delivery system as described in any of paragraphs (1) to (8) above, wherein the stent can be re-contained into the sheath until the stent is released from the engagement through breaking of the stent proximal portion fixing wire.
(10) The stent delivery system as described in any of paragraphs (1) to (9) above, wherein the stent proximal portion fixing wire is a thermoplastic resin fiber.

(11) The stent delivery system as described in any of paragraphs (1) to (10) above, wherein the shaft section has a proximal-side stopper which is located near the proximal end of a part where the stent is disposed and which restrains the stent from moving in the proximal direction.
(12) The stent delivery system as described in paragraph (11) above, wherein the proximal-side stopper is a spring-formed stopper wound around the shaft section.
(13) The stent delivery system as described in paragraph (12) above, wherein the stent proximal portion fixing wire extends in the direction of the stent by passing through a gap in a coil constituting the spring-formed stopper, from one end portion and the other end portion which are fixed to the shaft section.
(14) The stent delivery system as described in any of paragraphs (2) to (13) above, wherein the small holes formed in the stent for permitting the stent proximal portion fixing wire to pass therethrough has a low-friction inner surface or an easily releasable form for enhancing releasability of the wire.
(15) The stent delivery system as described in any of paragraphs (1) to (14) above, wherein the stent does not have any free end, due to a structure in which an apex or a portion near the apex, of a proximal-side bent portion, is joined to another linear element.
(16) The stent delivery system as described in any of paragraphs (1) to (15), wherein the stent includes a plurality of wave-shaped struts extending in the axial direction of the stent from one end side to the other end side of the stent and arranged in the circumferential direction of the stent, and one or more connecting struts for each interconnecting the wave-shaped struts which are adjacent to each other, the one or more connecting struts extending in the axial direction over a predetermined length, and, further, the wave-shaped struts each have end portions each joined to an end portion of the wave-shaped strut close thereto.
(17) The stent delivery system as described in paragraph (16) above, wherein the connecting struts are each curved in a circular arc shape.
(18) The stent delivery system as described in paragraph (16) or (17) above, wherein the stent has joint sections for joining each of one-end--side end portion and an the-other-end-side end portion of each wave-shaped strut to an end portion of another wave-shaped strut close to the each wave-shaped strut, and the joint section on one-end-side and the joint section on the-other-end-side differ from each other in combination of the wave-shaped struts which are joined to each other.

## Claims

1. A stent delivery system (1) comprising: a stent (10) which is formed to be substantially cylindrical in shape, is compressed toward a center axis thereof at the time of insertion into a living body, and is capable of expanding outward when placed in the living body, to be restored into a pre-compression shape thereof; a shaft section (3) having a guide wire lumen (61); and a sheath (2) having the stent (10) contained in a distal portion thereof, the stent (10) being located at a position which is on the shaft section (3) and near the distal end of the shaft section;
wherein the stent delivery system (1) comprises a stent proximal portion fixing wire (5) having one end portion (5a) and the other end portion (5b) fixed to the shaft section (3), and having an intermediate portion (5c) engaged with a proximal portion of the stent (10), and a breaking member (7) for breaking the stent proximal portion fixing wire (5) to release the stent (10) from the engagement.

2. The stent delivery system (1) according to claim 1, wherein the stent (10) is provided in a proximal portion thereof with a plurality of small holes (18) permitting the stent proximal portion fixing wire (5) to pass therethrough, the small holes (18) being provided in a substantially annular pattern, and the intermediate portion (5c) of the stent proximal portion fixing wire (5) is passed annularly through the plurality of small holes (18) in the stent (10).

3. The stent delivery system (1) according to claim 1, wherein the stent (10) has a plurality of proximal direction bent portions located at a proximal portion thereof, and the intermediate portion (5c) of the stent proximal portion fixing wire (5) is passed annularly through the plurality of proximal direction bent portions of the stent (10).

4. The stent delivery system (1) according to any of claims 1 to 3, wherein the shaft section (3) has a distal tube (31) having the guide wire lumen (61), and a shaft body (33) having a distal portion fixed to the proximal side of the distal tube (31), and the breaking member (7) is provided at the distal portion of the shaft body (33).

5. The stent delivery system (1) according to any of claims 1 to 4, wherein the stent (10) has a distal portion oriented toward the distal side of the sheath (2) and a proximal portion oriented toward the proximal side of the sheath (2); the stent (10) substantially does not have any bent free end at least projecting toward the proximal side, exclusively of the proximal portion; and, after exposure of the distal portion from the sheath (2), the exposed distal portion can be re-contained into the sheath (2) by moving the sheath (2).

6. The stent delivery system (1) according to any of claims 1 to 5, wherein the stent proximal portion fixing wire (5) is a heat-breaking stent proximal portion fixing wire, and the breaking member (7) is a heat-breaking member.

7. The stent delivery system (1) according to claim 6, wherein the heat-breaking member comprises a heat generating section (36) for breaking, an electric cable (64,65) having a distal portion connected to the heat generating section (36) and extending to a proximal portion of the shaft body (33), and a joint section (35) for joint to a power supply section, the joint section being connected to the electric cable (64,65) and formed at the proximal portion of the shaft body (33).

8. The stent delivery system (1) according to any of claims 1 to 7, wherein the shaft section (3) has a proximal-side opening of the guide wire lumen (61), the proximal-side opening opened at a side portion on the proximal side relative to a stent-containing part of the sheath (2); the sheath (2) has a sheath side hole (23) provided on the proximal side relative to the stent-containing part; and the guide wire can be inserted through the sheath side hole (23) and the proximal-side opening.

9. The stent delivery system (1) according to any of claims 1 to 8, wherein the stent (10) can be re-contained into the sheath (2) until the stent (10) is released from the engagement through breaking of the stent proximal portion fixing wire (5).

10. The stent delivery system (1) according to any of claims 1 to 9, wherein the stent proximal portion fixing wire (5) is a thermoplastic resin fiber.

11. The stent delivery system (1) according to any of claims 1 to 10, wherein the shaft section (3) has a proximal-side stopper (70) which is located near the proximal end of a part where the stent (10) is disposed and which restrains the stent (10) from moving in the proximal direction.

12. The stent delivery system (1) according to claim 11, wherein the proximal-side stopper (70) is a spring-formed stopper (70) wound around the shaft section (3).

13. The stent delivery system (1) according to claim 12, wherein the stent proximal portion fixing wire (5) extends in the direction of the stent (10) by passing through a gap in a coil constituting the spring-formed stopper (70), from one end portion (5a) and the other end portion (5b) which are fixed to the shaft section (3).

14. The stent delivery system (1) according to any of claims 2 to 13, wherein the small holes (18) formed in the stent (10) for permitting the stent proximal portion fixing wire (5) to pass therethrough has a low-friction inner surface or an easily releasable form for enhancing releasability of the wire.

15. The stent delivery system (1) according to any of claims 1 to 14, wherein the stent (10) does not have any free end, due to a structure in which an apex or a portion near the apex, of a proximal-side bent portion, is joined to another linear element.

## Patentansprüche

1. Stent-Zuführsystem (1) mit:
einem Stent (10), der mit einer im Wesentlichen zylindrischen Form geformt ist, der während der Einfügung in einen lebenden Körper zu seiner Mittelachse zusammengedrückt ist und der sich entfalten kann, wenn er in dem lebenden Körper platziert ist, um zu seiner vorkomprimierten Form wiederhergestellt zu werden; einem Schaftbereich (3) mit einem Führungsdrahtlumen (61); und einer Hülse (2), die den Stent (10) in ihrem distalen Abschnitt enthält, wobei sich der Stent (10) an einer Position befindet, die an dem Schaftbereich (3) und nahe dem distalen Ende des Schaftbereiches ist;
wobei das Stent-Zuführsystem (1) einen Draht (5) zum Befestigen eines proximalen Stentabschnitts, der einen Endabschnitt (5a) hat, wobei der andere Endabschnitt (5b) an dem Schaftbereich (3) befestigt ist, und der einen mittleren Abschnitt (5c) hat, der mit einem proximalen Abschnitt des Stents (10) im Eingriff ist, und ein Bruchelement (7) zum Brechen des Drahtes (5) zum Befestigen des proximalen Stentabschnitts aufweist, um den Stent (10) aus dem Eingriff zu lösen.

2. Stent-Zuführsystem (1) gemäß Anspruch 1, wobei der Stent (10) an seinem proximalen Abschnitt mit vielen kleinen Löchern (18) versehen ist, die es ermöglichen, dass der Draht (5) zum Befestigen des proximalen Stentabschnitts dort hindurch tritt, wobei die kleinen Löcher (18) im Wesentlichen in einem ringartigen Muster vorgesehen sind, und wobei der mittlere Abschnitt (5c) des Drahtes (5) zum Befestigen des proximalen Stentabschnitts ringartig durch die vielen kleinen Löcher (18) in dem Stent (10) hindurch tritt.

3. Stent-Zuführsystem (1) gemäß Anspruch 1, wobei der Stent (10) viele in proximaler Richtung gebogene Abschnitte hat, die sich an seinem proximalen Abschnitt befinden, und wobei der mittlere Abschnitt (5c) des Drahtes (5) zum Befestigen des proximalen Stentabschnitts ringartig durch die vielen, in proximaler Richtung gebogenen Abschnitte des Stents (10) hindurch tritt.

4. Stent-Zuführsystem (1) gemäß einem der Ansprüche 1 bis 3, wobei der Schaftbereich (3) eine distale Röhre (31) mit dem Führungsdrahtlumen (61) und einen Schaftkörper (33) mit einem distalen Abschnitt hat, der an der proximalen Seite der distalen Röhre (31) befestigt ist, und wobei das Bruchelement (7) an dem distalen Abschnitt des Schaftkörpers (33) vorgesehen ist.

5. Stent-Zuführsystem (1) gemäß einem der Ansprüche 1 bis 4, wobei der Stent (10) einen distalen Abschnitt, der zu der distalen Seite der Hülse (2) orientiert ist, und einen proximalen Abschnitt hat, der zu der proximalen Seite der Hülse (2) orientiert ist; wobei der Stent (10) im Wesentlichen kein gebogenes freies Ende hat, das zumindest zu der proximalen Seite vorsteht, und zwar ausschließlich vom proximalen Abschnitt; und, nachdem der distale Abschnitt aus der Hülse (2) freigelegt ist, der freigelegte distale Abschnitt erneut in der Hülse (2) aufgenommen werden kann, indem die Hülse (2) bewegt wird.

6. Stent-Zuführsystem (1) gemäß einem der Ansprüche 1 bis 5, wobei der Draht (5) zum Befestigen des proximalen Stentabschnitts ein durch Wärme brechender Draht zum Befestigen des proximalen Stentabschnitts ist, und das Bruchelement (7) ein durch Wärme brechendes Element ist.

7. Stent-Zuführsystem (1) gemäß Anspruch 6, wobei das durch Wärme brechende Element einen Wärme erzeugenden Bereich (36) zum Brechen, ein elektrisches Kabel (64, 65) mit einem distalen Abschnitt, der mit dem Wärme erzeugenden Bereich (36) verbunden ist und sich zu einem proximalen Abschnitt des Schaftkörpers (33) erstreckt, und einen Fügebereich (35) hat, um an einen Stromversorgungsbereich gefügt zu werden, wobei der Fügebereich mit dem elektrischen Kabel (64, 65) verbunden und an dem proximalen Abschnitt des Schaftkörpers (33) ausgebildet ist.

8. Stent-Zuführsystem (1) gemäß einem der Ansprüche 1 bis 7, wobei der Schaftbereich (3) eine Öffnung an der proximalen Seite des Führungsdrahtlumens (61) hat, wobei die Öffnung an der proximalen Seite an einem Seitenabschnitt an der proximalen Seite bezüglich eines Stent-Aufnahmeteils der Hülse (2) mündet; wobei die Hülse (2) ein hülsenseitiges Loch (23) hat, das an der proximalen Seite bezüglich des Stent-Aufnahmeteils vorgesehen ist; und wobei der Führungsdraht durch das hülsenseitige Loch (23) und die Öffnung an der proximalen Seite hindurch eingefügt werden kann.

9. Stent-Zuführsystem (1) gemäß einem der Ansprüche 1 bis 8, wobei der Stent (10) erneut in der Hülse (2) aufgenommen werden kann, bis der Stent (10) aus dem Eingriff durch das Brechen des Drahtes (5) zum Befestigen des proximalen Stentabschnitts gelöst wird.

10. Stent-Zuführsystem (1) gemäß einem der Ansprüche 1 bis 9, wobei der Draht (5) zum Befestigen des proximalen Stentabschnitts eine thermoplastische Kunststofffaser ist.

11. Stent-Zuführsystem (1) gemäß einem der Ansprüche 1 bis 10, wobei der Schaftbereich (3) einen Stopper (70) an der proximalen Seite hat, der sich nahe dem proximalen Ende eines Teiles befindet, an dem der Stent (10) angeordnet ist, und der den Stent (10) von einer Bewegung in der proximalen Richtung abhält.

12. Stent-Zuführsystem (1) gemäß Anspruch 11, wobei der Stopper (70) an der proximalen Seite ein durch eine Feder gebildeter Stopper (70) ist, die um den Schaftbereich (3) gewickelt ist.

13. Stent-Zuführsystem (1) gemäß Anspruch 12, wobei sich der Draht (5) zum Befestigen des proximalen Stentabschnitts in der Richtung des Stents (10) erstreckt, indem er durch einen Spalt in einer Spule hindurch tritt, die den durch die Feder gebildeten Stopper (70) bildet, und zwar von einem Endabschnitt (5a) und dem anderen Endabschnitt (5b), die an dem Schaftbereich (3) befestigt sind.

14. Stent-Zuführsystem (1) gemäß einem der Ansprüche 2 bis 13, wobei die kleinen Löcher (18), die in dem Stent (10) ausgebildet sind, um dem Draht (5) zum Befestigen des proximalen Stentabschnitts zu ermöglichen, dort hindurch zu treten, eine Innenfläche mit geringer Reibung oder einer leicht lösbaren Form haben, um eine Lösbarkeit des Drahts zu erleichtern.

15. Stent-Zuführsystem (1) gemäß einem der Ansprüche 1 bis 14, wobei der Stent (10) aufgrund einer Struktur, bei der eine Spitze oder ein Abschnitt nahe der Spitze eines gebogenen Abschnitts an der proximalen Seite an einem anderen geraden Element gefügt ist, kein freies Ende hat.

## Revendications

1. Système (1) de pose d'endoprothèse vasculaire comprenant : une endoprothèse vasculaire (10) qui est formée pour avoir essentiellement une forme cylindrique, est comprimée vers son axe central au moment de l'insertion dans un corps vivant, et est capable de s'étendre vers l'extérieur lorsqu'elle est placée dans le corps vivant, pour être rétablie dans sa forme pré-comprimée ; une section d'arbre (3) ayant une lumière de fil de guidage (61) ; et une gaine (2) ayant l'endoprothèse vasculaire (10) contenue dans sa partie distale, l'endoprothèse vasculaire (10) étant située à une position qui se trouve sur la section d'arbre (3) et à proximité de l'extrémité distale de la section d'arbre ;
dans lequel le système (1) de pose d'endoprothèse vasculaire comprend un fil (5) de fixation de partie proximale d'endoprothèse vasculaire ayant une partie d'extrémité (5a) et l'autre partie d'extrémité (5b) fixées à la section d'arbre (3), et ayant une partie intermédiaire (5c) engagée avec une partie proximale de l'endoprothèse vasculaire (10), et un élément de rupture (7) pour rompre le fil (5) de fixation de partie proximale d'endoprothèse vasculaire afin de libérer l'endoprothèse vasculaire (10) de l'engagement.

2. Système (1) de pose d'endoprothèse vasculaire selon la revendication 1, dans lequel l'endoprothèse vasculaire (10) est pourvue, dans sa partie proximale, d'une pluralité de petits trous (18) permettant au fil (5) de fixation de partie proximale d'endoprothèse vasculaire de passer à travers ceux-ci, les petits trous (18) étant prévus selon une configuration essentiellement annulaire, et la partie intermédiaire (5c) du fil (5) de fixation de partie proximale d'endoprothèse vasculaire est passée de manière annulaire à travers la pluralité de petits trous (18) dans l'endoprothèse vasculaire (10).

3. Système (1) de pose d'endoprothèse vasculaire selon la revendication 1, dans lequel l'endoprothèse vasculaire (10) comprend une pluralité de parties courbées de direction proximale situées au niveau de sa partie proximale, et la partie intermédiaire (5c) du fil (5) de fixation de partie proximale d'endoprothèse vasculaire est passée de manière annulaire à travers la pluralité de parties courbées de direction proximale de l'endoprothèse vasculaire (10).

4. Système (1) de pose d'endoprothèse vasculaire selon l'une des revendications 1 à 3, dans lequel la section d'arbre (3) comprend un tube distal (31) ayant la lumière de fil de guidage (61), et un corps d'arbre (33) ayant une partie distale fixée au côté proximal du tube distal (31), et l'élément de rupture (7) est prévu au niveau de la partie distale du corps d'arbre (33).

5. Système (1) de pose d'endoprothèse vasculaire selon l'une des revendications 1 à 4, dans lequel l'endoprothèse vasculaire (10) comprend une partie distale orientée vers le côté distal de la gaine (2) et une partie proximale orientée vers le côté proximal de la gaine (2) ; l'endoprothèse vasculaire (10) n'a essentiellement aucune extrémité libre courbée au moins faisant saillie vers le côté proximal, exclusivement de la partie proximale ; et, après exposition de la partie distale depuis la gaine (2), la partie distale exposée peut être à nouveau contenue dans la gaine (2) en déplaçant cette dernière.

6. Système (1) de pose d'endoprothèse vasculaire selon l'une des revendications 1 à 5, dans lequel le fil (5) de fixation de partie proximale d'endoprothèse vasculaire est un fil de fixation de partie proximale d'endoprothèse vasculaire à rupture thermique et l'élément de rupture (7) est un élément de rupture thermique.

7. Système (1) de pose d'endoprothèse vasculaire selon la revendication 6, dans lequel l'élément de rupture thermique comprend une section de génération de chaleur (36) permettant la rupture, un câble électrique (64, 65) ayant une partie distale reliée à la section de génération de chaleur (36) et s'étendant vers une partie proximale du corps d'arbre (33), et une section de raccordement (35) permettant le raccordement à une section d'alimentation électrique, la section de raccordement étant reliée au câble électrique (64, 65) et formée au niveau de la partie proximale du corps d'arbre (33).

8. Système (1) de pose d'endoprothèse vasculaire selon l'une des revendications 1 à 7, dans lequel la section d'arbre (3) comprend une ouverture côté proximal de la lumière de fil de guidage (61), l'ouverture côté proximal étant ouverte au niveau d'une partie latérale sur le côté proximal par rapport à une partie contenant l'endoprothèse vasculaire de la gaine (2) ; la gaine (2) comprend un trou côté gaine (23) prévu sur le côté proximal par rapport à la partie contenant l'endoprothèse vasculaire ; et le fil de guidage peut être inséré à travers le trou côté gaine (23) et l'ouverture côté proximal.

9. Système (1) de pose d'endoprothèse vasculaire selon l'une des revendications 1 à 8, dans lequel l'endoprothèse vasculaire (10) peut être à nouveau contenue dans la gaine (2) jusqu'à libération de l'endoprothèse vasculaire (10) de l'engagement par la rupture du fil (5) de fixation de partie proximale d'endoprothèse vasculaire.

10. Système (1) de pose d'endoprothèse vasculaire selon l'une des revendications 1 à 9, dans lequel le fil (5) de fixation de partie proximale d'endoprothèse vasculaire est une fibre de résine thermoplastique.

11. Système (1) de pose d'endoprothèse vasculaire selon l'une des revendications 1 à 10, dans lequel la section d'arbre (3) comprend une butée côté proximal (70) qui est située à proximité de l'extrémité proximale d'une partie où l'endoprothèse vasculaire (10) est disposée et qui empêche le déplacement de l'endoprothèse vasculaire (10) dans la direction proximale.

12. Système (1) de pose d'endoprothèse vasculaire selon la revendication 11, dans lequel la butée côté proximal (70) est une butée (70) formée par un ressort enroulée autour de la section d'arbre (3).

13. Système (1) de pose d'endoprothèse vasculaire selon la revendication 12, dans lequel le fil (5) de fixation de partie proximale d'endoprothèse vasculaire s'étend dans la direction de l'endoprothèse vasculaire (10) en passant à travers un espace dans une bobine constituant la butée (70) formée par un ressort, depuis une partie d'extrémité (5a) et l'autre partie d'extrémité (5b) qui sont fixées à la section d'arbre (3).

14. Système (1) de pose d'endoprothèse vasculaire selon l'une des revendications 2 à 13, dans lequel les petits trous (18) formés dans l'endoprothèse vasculaire (10) pour permettre au fil (5) de fixation de partie proximale d'endoprothèse vasculaire de passer à travers ceux-ci comprennent une surface intérieure à faible coefficient de frottement ou une forme facilement libérable pour améliorer la capacité de libération du fil.

15. Système (1) de pose d'endoprothèse vasculaire selon l'une des revendications 1 à 14, dans lequel l'endoprothèse vasculaire (10) n'a aucune extrémité libre, en raison d'une structure dans laquelle un sommet ou une partie à proximité du sommet, d'une partie courbée côté proximal, est relié(e) à un autre élément linéaire.
